# EUROPEAN PATENT APPLICATION

(11) **EP 4 349 954 A1**
(43) Date of publication of application: **10.04.2024**
(21) Application number: 22199626.7
(22) Date of filing: 04.10.2022
(51) Int. Cl.: C12M 1/12, B01D 65/10, C12M 1/36, G01M 3/22, G01N 15/08

(54) **METHOD FOR PERFORMING AN INTEGRITY TEST ON A TESTING CONSUMABLE OF A BIOPROCESS INSTALLATION**

(71) Applicant: Sartorius Stedim Biotech GmbH, 37079 Göttingen (DE)
(72) Inventor: CHATTERJEE, Kirit, 37085 Göttingen (DE)
(74) Representative: Gottschald Patentanwälte Partnerschaft mbB

(57) **Abstract**

The invention relates to a method for performing an integrity test on a testing consumable (1) of a bioprocess installation using a test arrangement (2), wherein during a test routine (26), which is being performed by a test control unit (3) of the test arrangement (2), a predetermined test procedure is being applied to the testing consumable (1) via the test arrangement (2) and an integrity state is being derived from process data that have been collected during the test routine (26). It is proposed that during the test routine (26), early process data (27), that are being generated by a testing sensor arrangement (4) of the test arrangement (2) in an early stage of the test procedure, are being used to determine the integrity state of the testing consumable (1) by correlating in a correlating step (28) the early process data (27) to a system model (29) of a reference module (30), which reference module (30) emulates a, in particular integral, testing consumable (1) only in view of predetermined process data.

## Description

The present invention relates to a method for performing an integrity test on a testing consumable of a bioprocess installation according to the general part of claim 1, to a method for training a machine learning model according to the general part of claim 10, to a test control unit adapted for use in the first proposed method according to claim 18, to a training arrangement for performing the second proposed method according to claim 19 and to a data storage device with a trained machine learning model according to claim 20.

The term "bioprocess" presently represents any kind of biotechnological process, in particular biopharmaceutical processes. An example of such a bioprocess is the use of a bioreactor to cultivate microorganisms or mammalian cells under given conditions, wherein a cell broth is transferred from the bioreactor to a downstream process. Such a downstream process may be a separation of cells and supernatant via a filter. Before or after using such a filter, often, the filter has to be integrity tested to ensure its integrity. In particular, pre- and post-integrity testing is mandated by most regulatory authorities.

The method in question for performing an integrity test on a testing consumable of a bioprocess installation may be applied in various fields of biotechnology. The need for high efficiency in this field has been driven by the increasing demand for bioproducts, such as biopharmaceutical drugs. The efficiency in this sense is to be seen regarding not only the cost-effectiveness of the components to be used but also the controllability of the processes connected thereto. Integrity testing of consumables such as filters are an important, usually mandatory, but time-consuming part of many bioprocesses.

Nowadays, integrity tests are usually performed as follows: First, an integrity testing device pressurizes an external volume to a predetermined test pressure. The integrity testing device then maintains said test pressure for a predetermined stabilization time. Subsequently, after a measurement lasting for a predetermined testing time, the integrity test is evaluated to be a passed test, if the measured quantity stays below or above a predetermined threshold value or within a predetermined range. Depending on the test procedure being performed, this measured quantity may be at least one out of the group of the diffusion of air through a wetted filter membrane, the flow of water through a hydrophobic filter membrane, and/or the pressure at which the diffusion of air through a wetted filter membrane displays a disproportionate increase thus identifying the so-called "bubble point" of the filter.

The known prior art (WO 2016/030013 A1) is related to a method according to the general part of claim 1. During a test routine, which is being performed by a test control unit of a test arrangement, a predetermined test procedure is being applied to the testing consumable via the test arrangement. Prior to the invention in the mentioned prior art, this test procedure had to be run completely in order to derive an integrity state from process data that have been collected during the test routine. Nowadays, in many biotechnological production and/or quality control lines, such a method for performing an integrity test on a testing consumable still constitutes the bottleneck in terms of process efficiency. The resulting combination is of only restricted efficiency in view of process times and accuracy as well as in the controllability of the overall process. EP 2 425 886 A1 further shows an integrity testing device usable with the proposed method.

The mentioned prior art provides a solution for predicting the result of an integrity test during an early phase of the test procedure by analyzing early process data based on known early process data of integral consumables. However, deriving the necessary data to make this prediction is time-consuming itself.

It is an object of the present invention to provide a method for performing an integrity test on a testing consumable of a bioprocess installation, which increases accuracy as well as time-effectiveness of the integrity test with low effort.

The invention is based on the problem of improving the known method such that accuracy, as well as time-effectiveness of the integrity test, is increased with low effort.

The above-noted problem is solved for a method for performing an integrity test on a testing consumable with the features of the general part of claim 1 by the features of the characterizing part of claim 1.

One basic realization for the present invention is that an integrity test does not need to be necessarily performed completely. It has been found that an integrity state of a testing consumable can already be determined based on early process data by comparing this early process data to a system model describing early process data of known integral consumables. To be able to do this comparison, a high amount of data about integral consumables is necessary. For example, if the consumable is a filter, this means taking measurement of a high number of filters. This usually means having to wet a high number of filters, running long test procedures on the filters, drying them, etc.

It is particularly desirable to be able to use machine learning for the task of identifying integral consumables, particularly filters, from early process data as the process of identifying integral consumables has well-defined criteria, but even for different consumables regarded as integral, the early process data may deviate from each other. To train a machine learning model, the number of reference measurements need to be particularly large, making it very tedious and time-consuming to actually gather this data.

The main realization of the present invention is that such a system model can be derived from a reference module, which is not a "real" testing consumable. This reference module, for example a needle valve, is able to emulate a testing consumable for at least some process parameters, while for other process parameters the needle valve will not be able to emulate a testing consumable.. Here and preferably, the needle valve is an automated, preferably motorized, needle valve.

A reference procedure, in particular the test procedure, is applied to the reference module instead of testing consumables thereby generating process data for process parameters. The process parameters for which the reference module emulates a testing consumable are measured and then form generic early process data that can be used to derive a system model of, for example, integral filters..

The above is therefore facilitated by the use of said reference module, which is able to mimic an integral testing consumable, such as a filter setup in view of predetermined process data, in particular a fluid flow comparable to the leakage created by a testing consumable. The vessel volume used in the reference module can be changed to mimic various filter setups. By using said reference module, the time required to train the machine learning model can be significantly reduced as no "real" testing consumables are needed for training. This way, generation of the system model may be conducted much faster.

During the test routine of an integrity test on a testing consumable, the measured early process data is correlated to the mimicked predetermined early process data to determine the respective integrity state. Consequently, also during the test routine, the process times can be minimized.

Ultimately, reducing the times required to generate the system model and reducing the process times during the test routine, the total time required for performing an integrity test can be minimized.

In detail, it is proposed that during the test routine, early process data, that are being generated by a testing sensor arrangement of the test arrangement in an early stage of the test procedure, are being used to determine the integrity state of the testing consumable by correlating in a correlating step the early process data to a system model of a reference module, which reference module emulates a, in particular integral, testing consumable only in view of predetermined process data.

Claim 2 describes preferred components of the test arrangement. Such a test arrangement allows to perform an integrity test on a testing consumable with low effort and optimizes the manufacturing footprint. In particular, a reusable, preferably single-device, integrity testing device may be used for easy handling of the integrity test.

An embodiment according to claim 3 is directed to the steps of a preferred test routine. In one preferred embodiment, the usual test procedure is cut short, and the integrity state derived by the proposed method may be used as a final integrity state thereby deriving an integrity state with low effort. In particular, if the testing consumable is determined not to be integral, waiting for the rest of the integrity test may be considered a waste of time. If no failure of the integrity test is detected early it may be necessary to wait for the result of the test procedure.

In an embodiment according to claim 4, the sensor arrangement generates process data during some or all steps of the test procedure. The process data may, for example, additionally be used to determine a success or failure of the test procedure from later process data, in particular process data of the testing step. Furthermore, claim 4 gives a preferred definition of early process data.

Claim 5 is directed to a preferred way in which the reference module may have been used to derive the system model during a reference routine and therefore directly describes the preferred system model used in the proposed method.

The preferred embodiment according to claim 6 refers to the derivation of the system model from generic process data in the reference routine. In this preferred case, at least one process parameter of the early process data that emulates an integral testing consumable is measured by the testing sensor arrangement and the reference sensor arrangement alike.

Claim 7 gives preferred embodiments of the reference module. In particular, a needle valve and a calibrated leak have been found to be well usable as a replacement for real testing consumables, particularly filters. Both devices generate process data that emulates an integral testing consumable in many of the relevant aspects.

The preferred embodiment according to claim 8 refers to the system model, which is a trained machine learning model. This is in particular advantageous since the accuracy of the system model can be increased and the complexity of deriving the system model may be reduced.

In claim 9 a low complexity solution for the result of the correlation between the early process data and the system model is defined. In particular, a trained machine learning model can be used to classify the testing consumable into either integral or non-integral classes.

The preferred embodiment according to claim 10 refers to a preferred option regarding determining the integrity state, based on a measured, predetermined physical property of the testing consumable 1. For example, the machine learning model can be trained to recognize different filter sizes. If the machine learning model now recognizes a filter as having a flow higher than it actually has, the filter can be determined not to be integral.

The embodiments according to claim 11 refer to preferred options regarding the different possible test procedures being compatible with the proposed solution.

Another teaching according to claim 12, which is of equal importance, relates to a method for training a machine learning model.

All explanations given with regard to the proposed method for performing an integrity test are fully applicable. In particular, all features of the method for training the machine learning model may be features of the trained machine learning model and as such combined with the teachings of the first method. Both methods may be combined into a joint method and/or the features of the first method may influence the purpose of the second method and therefore also be descriptive of parts of the second method.

All explanations given with regard to the training of the machine learning model may, as far as applicable, also be used for generating the system model in general with or without using machine learning.

According to claim 13, the training arrangement may be concurrent to the testing arrangement in its main aspects. In this way, the generic early process data can be closer to data of actual testing consumables thereby enabling a better training of the machine learning model.

Claim 14 relates to the possibility of repeating the reference procedure to gather multiple measurements. The boundaries of the measurements may be changed between reference procedures, for example for each reference procedure or each number of reference procedures. The changes may affect a reference volume and/or a fluid pressure and/or a fluid temperature and/or a parametrization of the reference module and/or may be changes of the reference modules as such.

In claim 15, an especially preferred embodiment regarding the reference module is specified. These measures are particularly advantageous since they assure the comparability between the behavior of the testing consumable and the reference module and hence, the accuracy of the system model.

Claim 16 is directed to preferred parameters used to create generic process data. These aspects increase accuracy as well as reliability.

In claim 17, an especially preferred embodiment regarding the training of the machine learning model is specified. To train the machine learning model with data from actual measurements of testing consumables helps to increase the accuracy of the machine learning model in terms of reliably deriving an integrity state. This can be achieved either by mixing in data of testing consumables or by fine-tuning the machine learning model with said data. Both ways can be used with a low amount of data of testing consumables thereby increasing the accuracy at low time effort and cost.

The preferred embodiment according to claim 18 connects the method for training a machine learning model and the method for performing an integrity test on a testing consumable.

Another teaching according to claim 19, which is of equal importance, relates to a test control unit adapted for use in the proposed first method.

All explanations given with regard to the proposed method for performing an integrity test and to the proposed method for training a machine learning model are fully applicable.

Another teaching according to claim 20, which is of equal importance, relates to a training arrangement for performing the proposed second method.

All explanations given with regard to the proposed method for performing an integrity test and to the proposed method for training a machine learning model and to the test control unit are fully applicable.

Another teaching according to claim 21, which is of equal importance, relates to a data storage device with a trained machine learning model having been derived by the proposed second method.

All explanations given with regard to the proposed method for performing an integrity test and to the proposed method for training a machine learning model, to the test control unit, and to the training arrangement are fully applicable.

In the following, embodiments of the invention are explained with respect to the drawing. The drawing shows in
- Fig. 1,: a test arrangement for use in the proposed method,
- Fig. 2,: a usual pressure over time curve as may be expected from an integral testing consumable (solid line) and two examples of curves for non-integral testing consumables (dotted lines),
- Fig. 3,: a flow diagram of the proposed method,
- Fig. 4,: a proposed training arrangement and
- Fig. 5,: a proposed training routine for training a machine learning model.

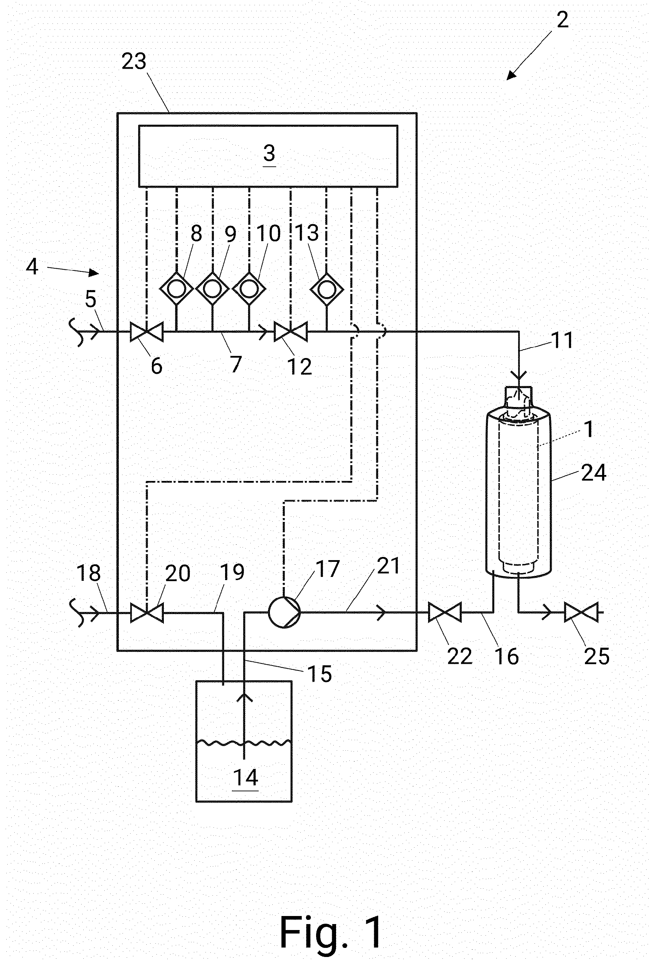

As shown in fig. 1, the proposed method for performing an integrity test on a testing consumable 1 of a bioprocess installation uses a test arrangement 2. The test arrangement 2 comprises a test control unit 3 and a testing sensor arrangement 4. For integrity testing, a predetermined test procedure is applied to the testing consumable 1 via the test arrangement 2. The test procedure may be applied automatically and/or manually.

Fig. 1 shows a preferred embodiment of the test arrangement 2 on which a preferred embodiment of the proposed method can be explained. The test arrangement 2 may comprise a test gas inlet 5, for supplying a test gas such as air, nitrogen or helium to the test arrangement 2. During an integrity test, the test control unit 3 opens a first valve 6, which is preferably designed as a proportional valve. The first valve 6 is used to control the flow rate or mass flow of the test gas through a gas fluid line 7 of the test arrangement 2. It is particularly preferred that the gas fluid line 7 comprises a first pressure sensor 8 to determine the pressure of the test gas, a temperature sensor 9 to determine the temperature of the test gas, and/or a flow sensor 10 to determine the flow rate of the test gas. Preferably the gas fluid line 7 comprises all of said sensors. The sensors of the testing sensor arrangement 4 produce process data, which are provided to the test control unit 3.

The gas fluid line 7 preferably connects the test gas inlet 5 of the test arrangement 2 with a test gas outlet 11, which leads to the testing consumable 1, such as a filter to be tested. Exemplarily, to be able to completely shut off the test gas supply to the test gas outlet 11 to perform the integrity test, in one instance a pressure decay test, the fluid line further comprises a second valve 12, preferably designed as shut-off valve. The gas fluid line 7 further comprises a second pressure sensor 13 downstream of the second valve 12 to determine the change of pressure of the test gas during the integrity test.

In order to perform the integrity test of the testing consumable 1 and depending on the test to be performed, a liquid may be provided. The liquid can be provided in a liquid container 14, which can be connected to a liquid supply 15 of the test arrangement 2. In order to transport the liquid from the liquid container 14 to the liquid outlet 16, the test arrangement 2 can comprise a liquid pump 17, in particular a peristaltic pump, which can be arranged downstream of the liquid supply 15. Alternatively, to a pump, the test arrangement 2 may comprise a second test gas inlet 18, which is connected to a second fluid line 19 with a third valve 20, preferably designed as proportional valve. The second fluid line 19 ends within the liquid container 14. This third valve 20 is used to control the flow rate or mass flow of the test gas through said fluid line of the test arrangement 2 in order to convey the liquid. Fig. 1 shows both alternatives in one figure only schematically.

Thereby, the liquid is conveyed from the liquid supply 15 through a third fluid line 21 via a fourth valve 22 to a liquid outlet 16, which leads to the testing consumable 1, such as a filter to be tested. The fourth valve 22 can be an on/off valve, a proportional valve, or a proportional control valve.

The described components may be gathered inside a corresponding integrity testing device 23, which preferably has a single housing containing the described components as far as they are present. However, all explanations given with regard to a single housing or single device integrity testing device 23 may also apply to an integrity testing arrangement 2in general. While the use of an above noted, compact integrity testing device is preferred in terms of easy handling, alternatively, the proposed method steps may be performed with one or more discrete components, such as a pressure generator or the like.

The testing consumable 1 is placed inside an external volume 24 also shown in fig. 1. Downstream of the external volume 24 a fifth valve 25 is shown.

The term "external volume" means any housing, cartridge or the like comprising a predetermined volume, in which a testing consumable 1 is placed. Hence, the external volume 24 may refer to an additional housing, cartridge or the like, in which a testing consumable 1 can be placed (Fig. 1), or, the external volume may 24 refer to a housing, in which the testing consumable 1 was placed by the manufacturer. According to this preferred embodiment the external volume 24 is already part of the testing consumable 1 (not shown).

Eventually, an integrity state is being derived from process data that have been collected during the test routine 26 by the testing sensor arrangement 4.

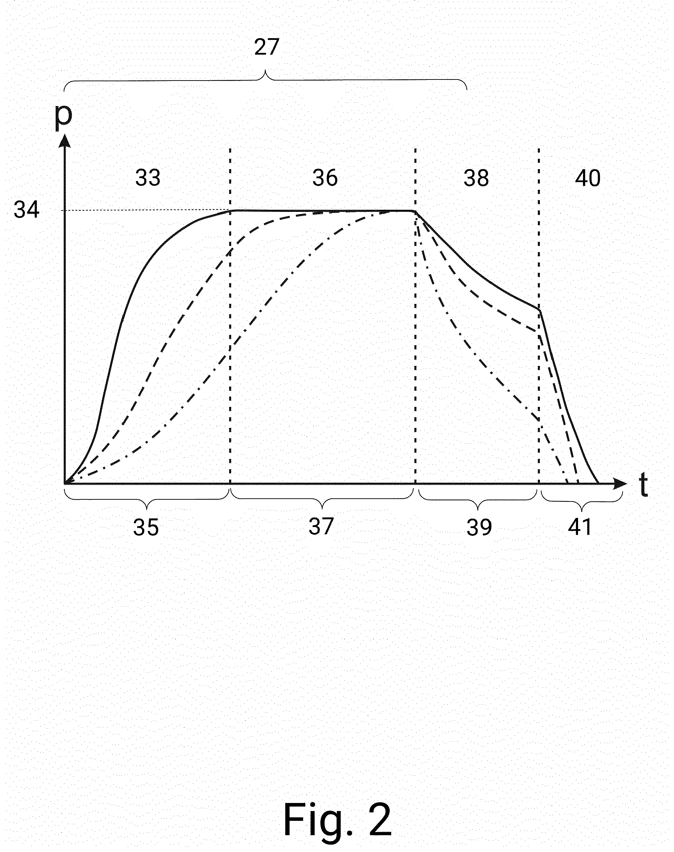

Fig. 2 shows exemplary pressure overtime curves for an integral testing consumable 1 (solid line) and for non-integral testing consumables 1 (dotted lines).

Proposed in detail is a method for performing an integrity test on a testing consumable 1 of a bioprocess installation using a test arrangement 2, wherein during a test routine 26, which is being performed by a test control unit 3 of the test arrangement 2, a predetermined test procedure is being applied to the testing consumable 1 via the test arrangement 2 and an integrity state is being derived from process data that have been collected during the test routine 26.

The proposed method for performing an integrity test on a testing consumable 1 of a bioprocess installation is preferably assigned to the quality control phase of a testing consumable 1 production line (not shown) and/or the testing before and/or after a bioprocess using the testing consumable 1.

In general, integrity tests are currently used during the production of testing consumables 1 and/or during the production of pharmaceutical or biopharmaceutical drugs, where the use of testing consumables 1 is required. As mentioned above, the integrity testing of the testing consumables 1 pre- and post-use is mandated by most regulatory authorities. Such testing consumables 1 are preferably filters, bags, or the like, further preferably designed for single use. Integrity testing of filters etc. is a fundamental requirement for critical filtration applications in the biotechnological and biopharmaceutical industries. FDA regulations demand integrity tests for all kinds of filters that are used for the production of sterile solutions, such as parenteralia. Additionally, official regulations require that the respective documents are added to each production protocol of any batch of produced filters and/or of bioproducts, such as biopharmaceutical drugs, which are produced using such filters.

The term "during a test routine" implies that the test procedure is at least partially carried out at the same time as the test routine 26, as far as necessary to collect the process data. However, it does neither necessarily imply that the test procedure runs during the entire test routine 26, nor that the test procedure is carried out by the test control unit 3. In particular, the test procedure may be completely or partially carried out by an operator, even though in the described embodiment it is carried out automatically. Data processing can be performed subsequently to the test procedure in the rest routine. The suggested test control unit 3 may be a local control unit or may include cloud services provided by a cloud server alternatively or in addition to local hardware. The term "control unit" should therefore be understood broadly.

Here and preferably the integrity state is being derived by the test control unit 3 during the test routine 26. Alternatively, it may also be derived in a later and/or external data analysis step by a different computation unit or the like.

Essential is that during the test routine 26, early process data 27, that are being generated by a testing sensor arrangement 4 of the test arrangement 2 in an early stage of the test procedure, are being used to determine the integrity state of the testing consumable 1 by correlating in a correlating step 28 the early process data 27 to a system model 29 of a reference module 30, which reference module 30 emulates a, in particular integral, testing consumable 1 only in view of predetermined process data.

Early process data is any data that has been generated prior to a point in time when a conventional testing procedure would be advanced far enough to reach a conclusion on the integrity test. For a multi step test procedure, those early process data may be defined as those data, that are being collected during a predefined step of the test procedure, wherein the steps are being distinct from each other, each serving different physical purposes.

The term "correlating" does not imply correlation as the mathematical concept but relates to any concept where the early process data 27 is input into the system model 29, the system model 29 is applied to the input data or otherwise used to determine the integrity state.

The reference module 30 is a device or arrangement of devices that is usable to emulate a testing consumable 1 regarding some process parameters. It can be used efficiently to gather data about the reaction of integral testing consumables 1 to certain test procedures without having to do laborious experiments with real testing consumables 1. While the present example is about emulating integral testing consumables 1, all explanations given may alternatively or in addition apply to non-integral testing consumables 1. Therefore, the system model 29 may be a system model of integral and/or non-integral testing consumables 1.

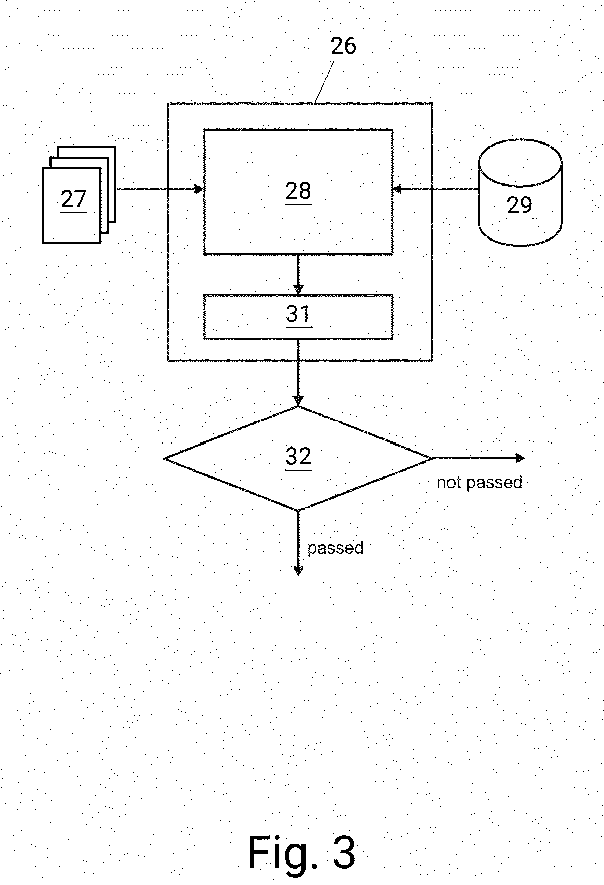

The test routine 26 is shown in fig. 3 as an example. After the transmittal of the early process data 27 from the testing sensor arrangement 4 to the test control unit 3, in a correlating step 28, the test control unit 3 correlates the early process data 27 to the system model 29. In a classification step 31, the integrity class of the integrity state is derived, preferably by a machine learning model as the system model 29. Depending on the derived integrity state, which is checked in a checking step 32, the testing consumable 1 is considered to have passed or not passed the integrity test. The correlating step 28 and the classification step 31 may be repeated continuously or may be initiated after a certain amount of time when the early process data 27 has been gathered completely.

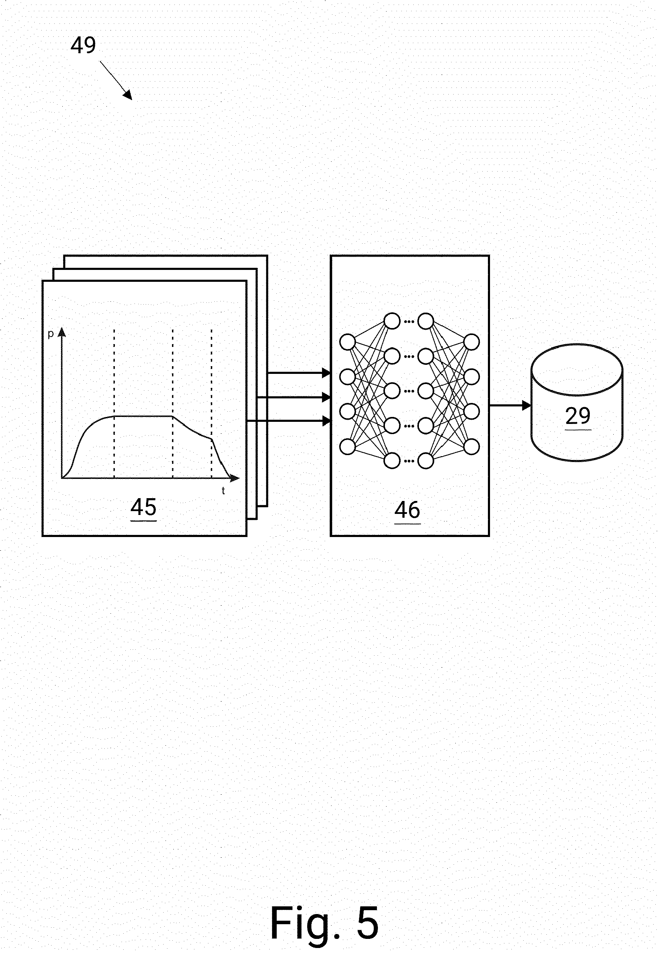

The machine learning model has been trained as part of the proposed first method or is being trained as part of the proposed second method. The concept of the training is shown in fig. 5. According to this, the training is preferably based on an unsupervised one-class classification approach. Presently, two classes, "integral" and "non-integral" should be detected. However, the proposed training data is mainly or only training data relating to integral testing consumables 1. To avoid having to gather data on non-integral testing consumables 1 somehow, a one-class classification model is used. The model is trained to learn to determine a single class and the second class is derived by detecting major deviations from the learned class. For example, a state vector machine may be used. An unsupervised approach is usable particularly well because it may already be known that all data relate to integral testing consumables 1. On the other hand, that knowledge can be used to simply tag all data as integral and use a supervised learning approach. Of course, data for non-integral testing consumables 1 can be used additionally.

According to one embodiment, it is proposed, that the test arrangement 2 comprises an integrity testing device 23 for performing the test procedure as shown in fig. 1. The integrity testing device 23 preferably comprises the test control unit 3. As mentioned already, the test arrangement 2 further may comprise an external volume 24 located downstream of the integrity testing device 23 in which the testing consumable 1 can be placed. The integrity testing device 23 and the external volume 24 are fluidically connected.

Here and preferably the integrity testing device 23 performs the test procedure by creating at least one predetermined process parameter in the test arrangement 2, in particular by creating a predetermined fluid pressure in the external volume 24. The external volume 24 may be designed as a multi or single-use housing. Here, the integrity testing device 23 is connected via a fluid line to the external volume 24 directly.

As shown in fig. 2 it is preferred, that, in the test procedure in a pressurization step 33, the integrity testing device 23 pressurizes the external volume 24 with a fluid to a predetermined test pressure 34 for a pressurization time 35 and that in the test procedure in a stabilization step 36 the integrity testing device 23 maintains said predetermined test pressure 34 for a stabilization time 37. As mentioned above, instead of the integrity testing device 23 also a pressure regulator or the like may be used to pressurize the external volume 24 to a predetermined test pressure 34 for a pressurization time 35 and the subsequent stabilization step 36.

These steps may be the steps of a conventional test procedure. The conventional test procedure steps may be followed further, or the test procedure may stop here to save time. In one embodiment, in the test procedure in a testing step 38 the integrity testing device 23 monitors a change of said maintained test pressure 34 for a testing time 39 and in the test procedure in a venting step 40 the integrity testing device 23 vents the test arrangement 2 for a venting time 41.

In another embodiment, the test procedure ends after the pressurization step 33 or the stabilization step 36. Further, both alternatives may be usable depending on the result of the checking step 32. Preferably, the test procedure ends if the testing consumable 1 is determined not to be integral, and/or the test procedure does not end if the testing consumable 1 is not determined not to be integral.

According to one embodiment, it is proposed that during one or more of these steps, preferably during all of these steps, the testing sensor arrangement 4 generates process data, preferably, that during the pressurization step 33 and/or the stabilization step 36 the testing sensor arrangement 4 generates the early process data 27.

The data generated this way is sent from the testing sensor arrangement 4 to the test control unit 3. The testing sensor arrangement 4 and/or the test control unit 3 may be part of the integrity testing device 23.

Looking now towards the generation of the system model 29, it may be the case that, during a reference routine, which has been performed by a reference control unit 42 of a training arrangement 43, a predetermined reference procedure has been applied to the reference module 30 via the training arrangement 43. These steps describe the properties of the system model 29. Alternatively, these steps may be part of the proposed method. Therefore, the reference routine may be performed, and the reference procedure may be applied.

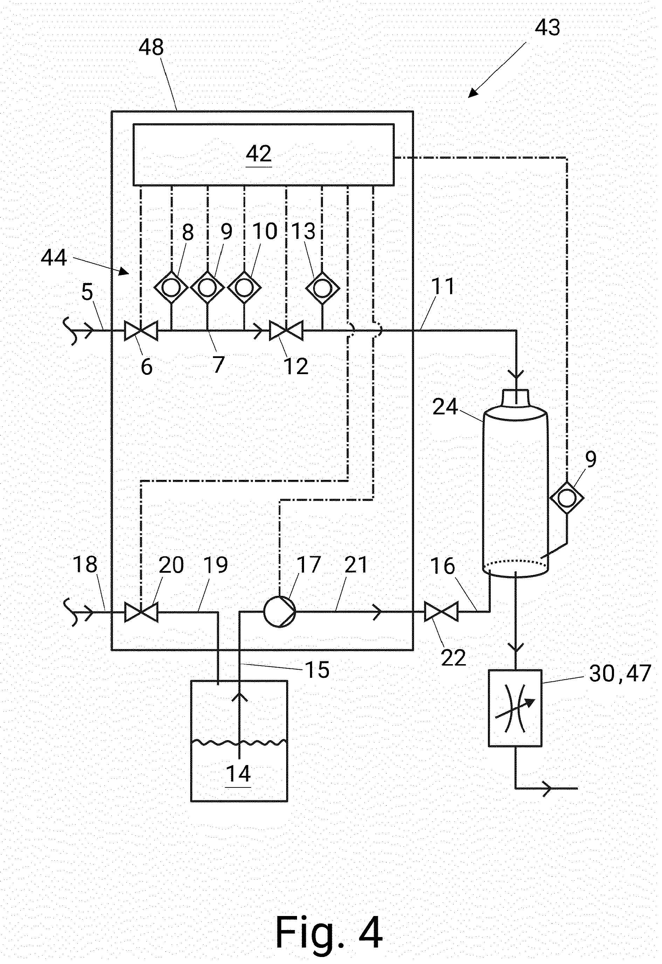

The training arrangement 43 comprises, meaning it may comprise now or have comprised a reference sensor arrangement 44. During the reference routine, the reference sensor arrangement 44 of the training arrangement 43 generates or generated generic early process data 45, and a training control unit 46 derived or derives the system model 29 from the generic early process data 45 in the reference routine. Fig. 4 shows the training arrangement 43.

The training control unit 46 may be the reference control unit 42 or a cloud service or the like.

Preferably, the testing sensor arrangement 4 and the reference sensor arrangement 44 are identical in construction and/or measure the same process parameters as far as these are used in the correlating step 28. Moreover, the testing arrangement 2 and the training arrangement 43 are preferably identical in construction. In a first step, this enables generating generic process data by using the training arrangement 43 with the reference sensor arrangement 44, which allows deriving the system model 29. In a second step, this enables generating process data by using the testing arrangement 2 with the testing sensor arrangement 4. These alike measurements form the basis for the correlating step 28, wherein the process data are correlated to the system model 29. Owing to the structural similarities between the testing arrangement 2 and the training arrangement 43, the same numerals have been used for the components not described again.

The generic early process data 45 may therefore be similar in the relevant aspects to the early process data 27.

According to one embodiment, it is proposed, that the early process data 27 represent at least one parameter being reflective of the integrity state of the testing consumable 1, that the generic early process data 45 represent at least the same parameter, and that for the at least one parameter the reference module 30 emulates a, in particular integral, testing consumable 1.

This at least one parameter measured in the testing arrangement 2 and the training arrangement 43 comprises one or more parameters out of the group of fluid pressure, amount of substance of the fluid, temperature of the fluid and/or flow rate of the fluid, preferably volumetric flow rate of the fluid. As shown in fig. 1, the fluid here and preferably is the test gas.

As already mentioned the reference module 30 may be of a different structure than the testing consumable 1. In detail, preferably the reference module 30 comprises a valve, preferably a needle valve 47, and/or, the reference module 30 comprises a calibrated leak (not shown).

As shown in fig. 4, the training arrangement 43 may comprise an external volume 24, even though the needle valve 47 is located downstream of the external volume 24. This however further helps to have similar physical characteristics of the test arrangement 2 and the training arrangement 43. The presence of the external volume 24 also allows mimicking different testing consumables 1 by adjusting the volume of the external volume 24. The volume of the external volume 24 may for example be adjusted by using an external volume 24 with a different geometry. The training arrangement 43 may comprise additionally or alternatively to the temperature sensor 9 of the parameter regulating device 48 a temperature sensor 9 in or at the external volume 24.

The system model 29 describes the reference module 30 in a way, that allows to determine the integrity state of a testing consumable 1 by correlating its early process data to the system model 29. The system model 29 may for example be a trained machine learning model, a statistical model or an analytical model.

According to the preferred embodiment, it is proposed, that the system model 29 is an above noted trained machine learning model, and that the machine learning model is applied by the test control unit 3 in order to determine the integrity state of the testing consumable 1 from the early process data 27.

Alternatively, as also noted above, the system model 29 is a statistical model or an analytical model.

The term "analytical model" means a quantitative model, which is used to answer a specific question. Its main goal is to provide a closed formula for specific characteristics. Hence, analytical models are mathematical models that have a closed-form solution, i.e., the solution to the equations used to describe changes in a system can be expressed as a mathematical analytic function.

The term "statistical model" means a mathematical model, in which some or all of the input data have some randomness, for example as expressed by a probability distribution, so that for a given set of input data the output is not reproducible but is described by a probability distribution. The output ensemble is obtained by running the model many times with a new input value sampled from the probability distribution each time the model is run.

Here and preferably the integrity state is represented by an integrity class out of a predetermined group of integrity classes. In the test routine 26, the integrity state is assigned one of the integrity classes. Preferably, the predetermined group of integrity classes only includes the integrity classes "integral" and "non-integral".

According to fig. 5 and as already mentioned, the machine learning model is preferably trained by a "one-class-classification"-training. This means, that the machine learning model is trained with data of a multitude of known integral testing consumables 1, in this case emulated by the reference module 30. Thereby, the model is able to recognize with increasing accuracy, whether a testing consumable 1 is integral. This trained machine learning model preferably forms the basis for deriving the system model 29 or is the system model 29.

Alternatively, the machine learning model is applied by the test control unit 3 in order to determine a predetermined physical property of the testing consumable 1, in particular a filter size of the filter, and the integrity state is being derived from the predetermined physical property.

The machine learning model may for example be trained to detect different filter sizes in the same or varying test setups and/or test environments like different temperatures. The integrity testing may work as follows. A filter with an exemplary flow rate of 10 ml/min may be tested. The early process data 27 is measured and fed into the machine learning model. The machine learning model, exemplarily trained with data of reference modules 30 for 10 ml/min, 12,5 ml/min and 15 ml/min then determines which flow rate the filter has. If the flow rate is determined to be 10 ml/min, the filer is integral. If the flow rate is determined to be 12,5 ml/min or 15 ml/min, the filter is not integral. Of course, the machine learning model may be trained to detect a small or a large number of different flow rates, for example at least three different flow rates, at least 10 different flow rates or at least 50 different flow rates. In particular, the flow rates may be based on a training arrangement 43 that is essentially identical for the different flow rates. Generating data for training the machine learning model for those different flow rates is greatly eased by using the reference module 30.

Accordingly, the testing consumable 1 may be determined to be integral if the determined physical property matches a nominal physical property and/or determined not to be integral if the determined physical property does not match a nominal physical property.

According to one embodiment, it is proposed, that the test procedure is a test procedure out of the group of diffusion test, in particular forward flow test and multipoint diffusion test, and bubble point test. Here it should be noted that different test procedures (e.g., forward flow test and bubble point test) may also be combined.

Alternatively, further test procedures are applicable, in particular a water intrusion test, a water flow test or the like. Preferably, all test procedures are applicable that are feasible for the used integrity testing device 23.

Another teaching, which is of equal importance relates to a method for training a machine learning model usable as the system model 29 for the proposed method for performing an integrity test using a training arrangement 43.

During a reference routine, which is being performed by a reference control unit 42 of the training arrangement 43, a predetermined reference procedure is being applied to the reference module 30 via the training arrangement 43. The training arrangement 43 comprises a reference sensor arrangement 44, wherein generic early process data 45 are generated by the reference sensor arrangement 44 during the reference routine.

During a training routine 49, which is being performed by a training control unit 46 of the training arrangement 43, the generic early process data 45 are used for training the machine learning model.

The training control unit 46 may be the reference control unit 42 or any different control unit. Here and preferably, the reference sensor arrangement 44 is identical to the testing sensor arrangement 4 and the predetermined reference procedure may be the predetermined testing procedure or may be or comprise the pressurization step 33 and/or the stabilization step 36.

All explanations given with regard to the proposed method for performing an integrity test are fully applicable.

According to one embodiment, it is proposed that the training arrangement 43 comprises a parameter regulating device 48 for performing the reference procedure.

The parameter regulating device 48 may be provided by an integrity testing device 23 also used for the proposed first method. While the use of an integrity testing device 23 is preferred, as noted with regard to the first teaching, alternatively, the proposed method steps may be performed with one or more discrete components, such as a pressure generator or the like, performing at least the function of the parameter regulating device 48. Preferably, the same type of device is used for performing the first method and the second method. The parameter regulating device 48 may comprise the reference control unit 42 and/or the reference sensor arrangement 44.

As shown in fig. 4, the training arrangement 43 preferably further comprises an external volume 24 located downstream of the parameter regulating device 48. The training arrangement 43 comprises the reference module 30 located downstream of the parameter regulating device 48 and preferably downstream of the external volume 24.

In the preferred embodiment, the parameter regulating device 48 performs the reference procedure by creating at least one predetermined process parameter in the training arrangement 43, in particular, a predetermined fluid pressure at the reference module 30 and/or in the external volume 24.

The predetermined process parameter may be the same one as used in the test procedure for integrity testing.

In this case, in the external volume 24, there is no consumable and the external volume 24 is only there to better simulate the process parameters.

The parameter regulating device 48, the external volume 24, and the reference module 30, are fluidically connected.

Here it is proposed that the reference procedure and the reference routine are repeated a multitude of times, preferably at least 50 times, more preferably at least 200 times. Repetitions are advantageous for generating varying generic early process data 45. In the training routine 49, the varying generic early process data 45 are used for training the machine learning model. Preferably the reference module 30 is parameterized differently during at least some of the repetitions to emulate physical variations in integral testing consumables 1.

As described, here and preferably, the reference module 30, preferably a needle valve 47 or calibrated leak, creates a fluid flow mimicking the fluid flow of an integral testing consumable 1 at the training pressure value.

According to one embodiment, it is proposed, that in the training routine 49, the at least one parameter, preferably at least two parameters, further preferably all parameters measured by the testing sensor arrangement 4, in particular the fluid pressure, the amount of substance of the fluid, the temperature and/or the flow rate of the fluid, are used to create the generic early process data 45.

One interesting point is that synthetic data as proposed may not be as varying as data of testing consumables. Therefore, the training of the machine learning model may lead to a model that wrongly classifies some integral testing consumables 1 as not integral if they show variations not present in the generic early process data 45.

For that reason, it may be the case that the trained machine learning model is used as a pre-trained machine learning model. Following, during a fine-tuning routine, which is being performed by a training control unit 46 of the training arrangement 43, early process data of testing consumables 1, in particular of integral testing consumables 1, are used for fine-tuning the pre-trained machine learning model. The training control unit 46 may be the reference control unit 42 or a different control unit.

Additionally or alternatively the generic early process data 45 and early process data 27 of testing consumables 1 are mixed to obtain mixed early process data and during the training routine 49 the mixed early process data are used for training the machine learning model.

According to one embodiment, it is proposed, that the trained machine learning model is used in the proposed first method.

Another teaching, which is of equal importance relates to a test control unit 3 adapted for use in the proposed first method.

All explanations given with regard to the proposed method for performing an integrity test and to the proposed method for training a machine learning model are fully applicable.

Another teaching, which is of equal importance relates to a training arrangement 43 for performing the proposed second method.

All explanations given with regard to the proposed method for performing an integrity test and to the proposed method for training a machine learning model and to the test control unit 3 are fully applicable.

Another teaching, which is of equal importance relates to a data storage device with a trained machine learning model having been derived by the proposed second method.

The trained machine learning model may be adapted to be used as the system model 29 in the first proposed method.

All explanations given with regard to the proposed method for performing an integrity test and to the proposed method for training a machine learning model, to the test control unit 3, and to the training arrangement 43 are fully applicable.

### Reference numbers

- 1: testing consumable
- 2: test arrangement
- 3: test control unit
- 4: testing sensor arrangement
- 5: test gas inlet
- 6: first valve
- 7: gas fluid line
- 8: first pressure sensor
- 9: temperature sensor
- 10: flow sensor
- 11: test gas outlet
- 12: second valve
- 13: second pressure sensor
- 14: liquid container
- 15: liquid supply
- 16: liquid outlet
- 17: liquid pump
- 18: second test gas inlet
- 19: second fluid line
- 20: third valve
- 21: third fluid line
- 22: fourth valve
- 23: integrity testing device
- 24: external volume
- 25: fifth valve
- 26: test routine
- 27: early process data
- 28: correlating step
- 29: system model
- 30: reference module
- 31: classification step
- 32: checking step
- 33: pressurization step
- 34: predetermined test pressure
- 35: pressurization time
- 36: stabilization step
- 37: stabilization time
- 38: testing step
- 39: testing time
- 40: venting step
- 41: venting time
- 42: reference control unit
- 43: training arrangement
- 44: reference sensor arrangement
- 45: generic early process data
- 46: training control unit
- 47: needle valve
- 48: parameter regulating device
- 49: training routine

## Claims

1. Method for performing an integrity test on a testing consumable (1) of a bioprocess installation, using a test arrangement (2), wherein during a test routine (26), which is being performed by a test control unit (3) of the test arrangement (2), a predetermined test procedure is being applied to the testing consumable (1) via the test arrangement (2) and an integrity state is being derived from process data that have been collected during the test routine (26),
**characterized in**
**that** during the test routine (26), early process data (27), that are being generated by a testing sensor arrangement (4) of the test arrangement (2) in an early stage of the test procedure, are being used to determine the integrity state of the testing consumable (1) by correlating in a correlating step (28) the early process data (27) to a system model (29) of a reference module (30), wherein the reference module (30) emulates a, in particular integral, testing consumable (1) only in view of predetermined process data.

2. Method according to claim 1, **characterized in that** the test arrangement (2) comprises an integrity testing device (23) for performing the test procedure, wherein the integrity testing device (23) preferably comprises the test control unit (3), wherein the test arrangement (2) further comprises an external volume (24) located downstream of the integrity testing device (23) in which the testing consumable (1) is placed, that the integrity testing device (23) and the external volume (24) are fluidically connected, preferably, that the integrity testing device (23) performs the test procedure by creating at least one predetermined process parameter in the test arrangement (2), in particular fluid pressure in the external volume (24).

3. Method according to claim 2, **characterized in that**, in the test procedure in a pressurization step (33), the integrity testing device (23) pressurizes the external volume (24) with a fluid to a predetermined test pressure (34), that in the test procedure in a stabilization step (36) the integrity testing device (23) maintains said test pressure for a stabilization time (37), preferably, that in the test procedure in a testing step (38) the integrity testing device (23) monitors a change of said maintained test pressure for a testing time (39) and that in the test procedure in a venting step (40) the integrity testing device (23) vents the test arrangement (2) for a venting time (41), and/or, that the test procedure ends after the pressurization step (33) or the stabilization step (36), preferably, that the test procedure ends if the testing consumable (1) is determined not to be integral and/or the test procedure does not end if the testing consumable (1) is not determined not to be integral.

4. Method according to claim 3, **characterized in that** during one or more of these steps, preferably during all of these steps, the testing sensor arrangement (4) generates process data, preferably, that during the pressurization step (33) and/or the stabilization step (36) the testing sensor arrangement (4) generates the early process data (27).

5. Method according to any one of the preceding claims, **characterized in that**, during a reference routine, which has been performed by a reference control unit (42) of a training arrangement (43), a predetermined reference procedure has been applied to the reference module (30) via the training arrangement (43), that the training arrangement (43) comprises a reference sensor arrangement (44), that during the reference routine, the reference sensor arrangement (44) of the training arrangement (43) generated generic early process data (45) and that a training control unit (46) derived the system model (29) from the generic early process data (45) in the reference routine.

6. Method according to claim 5, **characterized in that** the early process data (27) represent at least one parameter being reflective of the integrity state of the testing consumable (1), that the generic early process data (45) represent at least the same parameter and that for the at least one parameter the reference module (30) emulates a, in particular integral, testing consumable (1), preferably, that the at least one parameter comprises one or more parameters out of the group of fluid pressure, amount of substance of the fluid, temperature of the fluid and/or flow rate of the fluid, preferably volumetric flow rate of the fluid.

7. Method according to any one of the preceding claims, **characterized in that** the reference module (30) is of a different structure than the testing consumable (1), preferably, that the reference module (30) comprises a valve, preferably a needle valve (47), and/or, that the reference module (30) comprises a calibrated leak.

8. Method according to any one of the preceding claims, **characterized in that** the system model (29) is a trained machine learning model, and that the machine learning model is applied by the test control unit (3) in order to determine the integrity state of the testing consumable (1) from the early process data (27).

9. Method according to any one of the preceding claims, **characterized in that** the integrity state is represented by an integrity class out of a predetermined group of integrity classes and that in the test routine (26), the integrity state is assigned one of the integrity classes, preferably, that the predetermined group of integrity classes only includes the integrity classes "integral" and "non-integral".

10. Method according to any one of claims 1 to 8, **characterized in that**, the machine learning model is applied by the test control unit (3) in order to determine a predetermined physical property of the testing consumable (1), in particular a filter size of the filter, and that the integrity state is being derived from the predetermined physical property, preferably, that the testing consumable (1) is determined to be integral if the determined physical property matches a nominal physical property and/or determined not to be integral if the determined physical property does not match a nominal physical property.

11. Method according to any one of the preceding claims, **characterized in that** the test procedure is a test procedure out of the group of diffusion test, in particular forward flow test and multipoint diffusion test, bubble point test, water intrusion test and water flow test.

12. Method for training a machine learning model usable as the system model (29) for a method for performing an integrity test according to any one of the preceding claims using a training arrangement (43),
wherein during a reference routine, which is being performed by a reference control unit (42) of the training arrangement (43), a predetermined reference procedure is being applied to the reference module (30) via the training arrangement (43),
wherein the training arrangement (43) comprises a reference sensor arrangement (44),
wherein generic early process data (45) are generated by the reference sensor arrangement (44) during the reference routine,
wherein during a training routine (49), which is being performed by a training control unit (46) of the training arrangement (43), the generic early process data (45) are used for training the machine learning model.

13. Method according to claim 12, **characterized in that** the training arrangement (43) comprises a parameter regulating device (48) for performing the reference procedure, wherein the parameter regulating device (48) preferably comprises the reference control unit (42), wherein the training arrangement (43) preferably further comprises an external volume (24) located downstream of the parameter regulating device (48), wherein the training arrangement (43) comprises a reference module (30) located downstream of the parameter regulating device (48) and preferably downstream of the external volume (24), preferably, that the parameter regulating device (48) performs the reference procedure by creating at least one predetermined process parameter in the training arrangement (43), in particular, fluid pressure at the reference module (30) and/or in the external volume (24).

14. Method according to claim 12 or 13, **characterized in that** the reference procedure and the reference routine are repeated a multitude of times, preferably at least 50 times, more preferably at least 200 times, generating varying generic early process data (45), that in the training routine (49) the varying generic process data are used for training the machine learning model, preferably, that the reference module (30) is parameterized differently during at least some of the repetitions to emulate physical variations in integral testing consumables (1).

15. Method according to any one of claims 12 to 14, **characterized in that** the reference module (30), preferably a needle valve (47) or calibrated leak, creates a fluid flow mimicking the fluid flow of an integral testing consumable (1) at the training pressure value.

16. Method according to any one of claims 12 to 15, **characterized in that** in the training routine (49), the at least one parameter, preferably at least two parameters, further preferably all parameters measured by the testing sensor arrangement (4), in particular the pressure, the amount of substance, the temperature and/or the flow rate, are used to create the generic early process data (45).

17. Method according to any one of the preceding claims 12 to 16, **characterized in that** the trained machine learning model is used as a pre-trained machine learning model, that, during a fine-tuning routine, which is being performed by a training control unit (46) of the training arrangement (43), early process data (27) of testing consumables (1), in particular of integral testing consumables (1), are used for fine-tuning the pre-trained machine learning model, and/or, that the generic early process data (45) and early process data (27) of testing consumables (1) are mixed to obtain mixed early process data (27) and during the training routine (49) the mixed early process data (27) are used for training the machine learning model.

18. Method according to any one of the claims 12 to 17, **characterized in that** the trained machine learning model is used in a method according to any one of the claims 1 to 11.

19. Test control unit adapted for use in the method according to any one of the claims 1 to 11.

20. Training arrangement for performing the method according to any one of the claims 12 to 17.

21. Data storage device with a trained machine learning model having been derived by a method according to claims 12 to 17.
